# EUROPEAN PATENT SPECIFICATION

(11) **EP 3 771 458 B1**
(45) Date of publication and mention of the grant of the patent: **06.05.2026**
(21) Application number: 19188807.2
(22) Date of filing: 29.07.2019
(51) Int. Cl.: A61G 13/02

(54) **REMOTE CONTROL FOR A MEDICAL APPARATUS, SYSTEM OF THE REMOTE CONTROL AND THE MEDICAL APPARATUS, AND METHOD FOR OPERATING THE MEDICAL APPARATUS**
FERNBEDIENUNG FÜR EINE MEDIZINISCHE VORRICHTUNG, SYSTEM AUS FERNBEDIENUNG UND MEDIZINISCHER VORRICHTUNG UND VERFAHREN ZUM BETRIEB DER MEDIZINISCHEN VORRICHTUNG
COMMANDE À DISTANCE POUR APPAREIL MÉDICAL, SYSTÈME DE COMMANDE À DISTANCE ET APPAREIL MÉDICAL ET PROCÉDÉ DE FONCTIONNEMENT DE L'APPAREIL MÉDICAL

(43) Date of publication of application: 03.02.2021
(73) Proprietor: Baxter Medical Systems GmbH + Co. KG, 07318 Saalfeld (DE)
(72) Inventor: ZEH, Leonard, Batesville, IN 47006 (US)
(74) Representative: Reddie & Grose LLP

(56) References cited:
- DE-A1- 19 751 329
- US-A1- 2009 126 115
- US-A1- 2012 118 718
- US-A1- 2015 245 965

## Description

The invention relates to a remote control for a medical apparatus, a system of the remote control and the medical apparatus, and a method for operating the medical apparatus, in particular, to a remote control for a medical apparatus having several movable segments driven in a controlled manner, a system of the remote control and the medical apparatus, and a method for operating the medical apparatus.

A remote control for a medical apparatus which controls motions of movable segments of the medical apparatus is known. For security against unintended use, the remote control usually includes at least one release switch and several switches for controlling motions in different directions of the respective movable segments. EP 2 455 959 A1 discloses such a remote control. However, in particular, in a case of an operating table as the medical apparatus, the number of the movable segments increases. Therefore, the number of the switches actuated by buttons or soft keys of a touch screen increases and the existing remote controls have the disadvantage that, due to the increased number of buttons or soft keys, an operation of the operating table became uncomfortable. Furthermore, an intuitive operation, in particular, an adjustment of a speed of the motion of the movable segments is not provided.

US 2009/126115 A1 provides a remote controller for an adjustable support apparatus for supporting a patient which includes an input device configured to accept control commands for adjusting a support apparatus for supporting a patient, at least one sensor configured to detect a pick-up of the remote controller by a user, and a transmitter device configured to transmit the control commands to the support apparatus for supporting a patient. The detection of the pick-up of the remote controller by the user activates the remote controller.

US 2015/245965 A1 provides operating units for a medical apparatus having a driven movable element comprising a fixing device for being fixed to the movable element, an actuating element, a signal processing device, at least one actuation sensor for detecting an actuation of the actuating element in at least one predetermined actuation axis, wherein the actuation sensor is adapted to supply an actuation signal depending on a direction of actuation in the actuation axis to the signal processing device and at least one direction sensor for outputting a direction signal. The signal processing device is adapted to align an actuation coordinate system according to the direction signal such that the direction of movement is equal to the direction of actuation.

The invention is based on the object to overcome the above disadvantages and to provide a remote control for a medical apparatus enabling a comfortable operation of the medical apparatus in an intuitive manner.

The object is achieved by a remote control according to claim 1, a system according to claim 7 and a method according to claim 15. Further developments of the invention are included in the dependent claims.

According to an aspect of the invention, a remote control for controlling a medical apparatus having at least one driven movable segment comprises a position sensor configured to detect a position and a motion of the remote control, at least one release switch; and at least one respective button or softkey for actuating the or each release switch. The remote control is configured to emit a control signal when the at least one release switch is actuated, by the respective button or softkey being pressed, to release a control of the motion of the at least one driven movable segment. The control signal is capable of controlling a motion of the at least one driven movable segment according to a motion of the remote control detected by the position sensor. The remote control is further configured to emit a stop command for stopping the motion of the at least one driven movable segment when the remote control is moved into a horizontal position.

By such a remote control, merely pressing of one button or one soft key for actuating the release switch is necessary for emitting a specific control signal since, after the release by the release switch, the control signal is then emitted due to the motion of the remote control itself.

**In** an advantageous implementation of the remote control, the remote control is configured to emit the control signal according to a direction of the motion of the remote control detected by the position sensor with respect to a position of the remote control when the at least one release switch is actuated to start the release of the control of the motion of the at least one driven movable segment.

When the control signal is emitted according to the direction of the motion of the remote control, an intuitive operation or motion of the movable segment is simply possible by moving the remote control according to a desired motion of the movable segment.

**In** a further advantageous implementation of the remote control, the remote control is configured to emit the control signal according to an amount of the motion of the remote control.

By emitting the control signal according to the amount of the motion of the remote control, a more precise command including characteristics of the motion of the remote control can be emitted for controlling the medical apparatus easily in an intuitive manner.

**In** a further advantageous implementation of the remote control, the remote control is configured to emit the control signal according to a speed of the motion of the remote control.

By emitting the control signal according to the speed of the motion of the remote control, also, a more precise command including characteristics of the motion can be emitted for comfortably controlling the medical apparatus in an intuitive manner.

**In** an advantageous implementation of the remote control, the position sensor comprises an inclinometer.

Inclinometers can be easily and economically obtained and, moreover, in some remote controls, an inclinometer is already included, e.g. for activating the remote control, so that the use for detecting the motion of the remote control for defining the control signal does not require further components of the remote control.

In a further advantageous implementation of the remote control, the position sensor comprises an acceleration sensor.

Also acceleration sensors can be easily and economically obtained and, moreover, in some remote controls, an acceleration sensor is already included, e.g. for activating the remote control, so that the use for detecting the motion of the remote control for defining the control signal does not require further components of the remote control.

According to a further aspect of the invention, a system of the remote control and the medical apparatus is provided. The remote control is configured to control the motion of the at least one driven movable segment of the medical apparatus by the control signal.

By the provision of this system, the medical apparatus can be comfortably operated without a need for searching an appropriate button or soft key for operating a specific motion of the movable segment of the medical apparatus.

In an advantageous implementation of the system, the remote control is configured to control a direction of the motion of the at least one driven movable segment according to a direction of the motion of the remote control with respect to a position of the remote control when the at least one release switch starts to release the control of the motion.

By this characteristic, the motion of the movable segment can be initiated intuitively so that the operation of the medical apparatus is facilitated.

In a further advantageous implementation of the system, the remote control is configured to control a speed of the motion of the at least one driven movable segment according to an amount of the motion of the remote control with respect to a position of the remote control when the at least one release switch starts to release the control of the motion, wherein the speed of the driven movable segment is the higher the larger the amount of the motion of the remote control is.

Due to this feature, a further characteristic of the motion of the movable segment can be determined in an easy and intuitive manner. Contrary to the existing remote controls, when possible anyway, it is not necessary to enter several menus to configure the speed of the motion.

In a further advantageous implementation of the system, the remote control is configured to control a speed of the motion of the at least one driven movable segment according to a speed of the motion of the remote control, wherein the speed of the motion of the at least one driven movable segment is the higher the higher the speed of the motion of the remote control is.

Due to this feature, a further characteristic of the motion of the movable segment can be alternatively or additionally determined in an easy and intuitive manner. Contrary to the existing remote controls, when possible anyway, it is not necessary to enter several menus to configure the speed of the motion.

In another advantageous implementation of the system, the remote control has an elongated shape, the motion of the remote control detected by the position sensor is at least one of a tilting around an axis of the remote control perpendicular to the elongated shape and a tilting around a longitudinal axis of the remote control parallel to the elongated shape.

In this implementation, easy and unambiguous motions of the remote control are used for determining the motion of the movable segment of the medical apparatus so that the intuitive operation of the medical apparatus is easily possible.

The remote control is configured to stop the motion of the driven movable segment when the remote control is moved into a horizontal position.

By this feature, stopping of the motion of the movable segment is easily and intuitively possible.

In an advantageous implementation of the system, the remote control comprises several release switches, the medical apparatus comprises at least one driven movable segment which performs several motions respectively driven by a driving device or the medical apparatus comprises several driven movable segments respectively driven by a driving device, and the several release switches are respectively assigned to a specific motion of one of the driven movable segments.

In such a system, several movable segments of the medical apparatus can be controlled in an easy and intuitive manner. On the one hand, if one movable segment performs several motions, for example, by one release switch, a height adjustment of a tabletop of an operating table can be released and, by another release switch, tilting of the tabletop around its longitudinal axis can be released, therefore, the several release switches release the control of the motions of one movable segment. On the other hand, if merely one motion is executed by one of the driven movable segment, the control of this motion is released, so that the several release switches release the motions of several driven movable segments.

In a further advantageous implementation of the system, the remote control comprises a communication interface configured to establish a communication to the medical apparatus in a wireless manner.

By the wireless communication between the remote control and the medical apparatus, a flexible use of the remote control without possibly disturbing medical personnel working at the medical apparatus is possible.

In another advantageous implementation of the system, the remote control comprises a communication interface having a connection cable configured to establish a communication to the medical apparatus in a wired manner.

By the wired communication, a safe and reliable communication without disturbances of obstacles or interference radiation is possible.

**In** an advantageous implementation of the system, the medical apparatus is configured as an operating table.

**In** particular, an operating table can comprise a large quantity of movable segments for adjusting the position of a patient in a surgical treatment. Moreover, there is a necessity for adjusting the movable segments during surgery. Therefore, the easy and intuitive operation of the operating table is a tremendous advantage.

According to another aspect of the invention, a method for operating a medical apparatus by means of a remote control comprises the steps: releasing a control of a motion of a specific one of driven movable segments of the medical apparatus by actuating a specific release switch of the remote control and controlling a motion of the specific one of the movable segments by moving the remote control with respect to a position of the remote control only while the specific release switch is being actuated, by pressing a respective button or softkey, to release the control of the motion of the specific one of the movable segments.

By the provision of this method, the movable segment of the medical apparatus can be operated without a need for searching an appropriate button or soft key for operating a specific motion of the movable segment of the medical apparatus.

**In** an advantageous implementation of the method, a direction of the motion of the specific one of the driven movable segments is defined by a direction of motion of the remote control around an axis perpendicular to an elongated shape of the remote control or around an axis parallel to the elongated shape of the remote control.

**In** this implementation of the method, easy and unambiguous motions of the remote control are used for determining the motion of the movable segment of the medical apparatus so that the intuitive operation of the medical apparatus is easily possible.

The method further comprises a step of emitting a stop command for stopping the motion of the specific one of the driven movable segments when the remote control is moved into a horizontal position.

By this implementation, stopping of the motion of the movable segment is easily and intuitively possible.

Below, the invention is elucidated by means of embodiments referring to the attached drawings.

In particular,
- Fig. 1: shows a system of a remote control in a plan view and an operating table in a side view;
- Fig. 2: shows a starting position and an end position of the remote control in a side view when the remote control is tilted around an axis perpendicular to an elongated shape of the remote control for controlling a motion;
- Fig. 3: shows another starting position and the end position of the remote control in the side view when the remote control is tilted around the axis perpendicular to the elongated shape of the remote control for controlling a motion;
- Fig. 4: shows a starting position and an end position of the remote control in a front view when the remote control is tilted around an axis parallel to an elongated shape of the remote control for controlling a motion;
- Fig. 5: shows another starting position and the end position of the remote control in front view when the remote control is tilted around the axis parallel to the elongated shape of the remote control for controlling a motion; and
- Fig. 6: shows a flow chart of a method according to the invention.

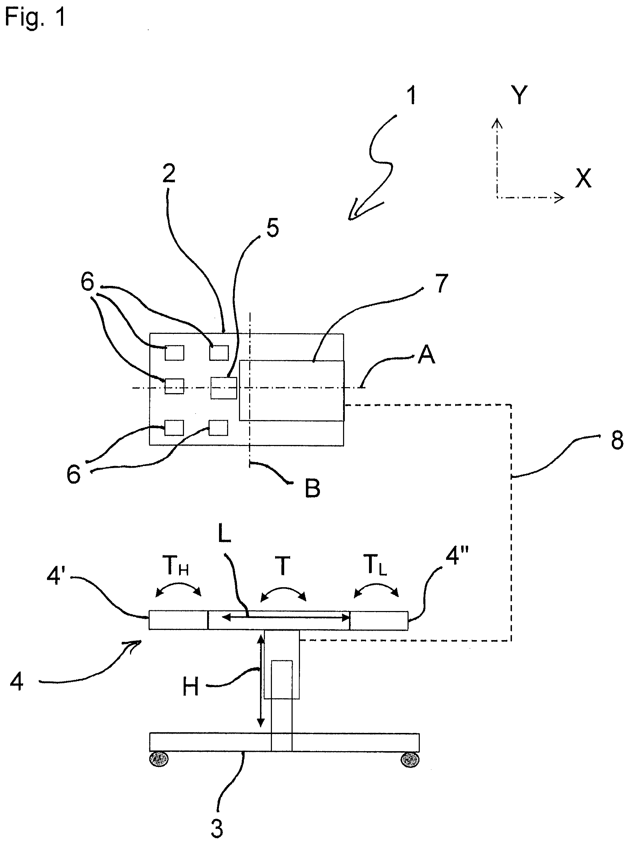

**Fig.** 1 shows a system 1 according to the invention including a remote control 2 in a plan view and an operating table as an embodiment of a medical apparatus 3 in a side view.

The medical apparatus 3 comprises several movable segments, namely, an entire tabletop 4, and a head plate 4' and a leg plate 4" of the tabletop 4. The movable segments 4, 4', 4" are respectively driven by at least one driving device (not shown). The tabletop 4 is moved by a first driving device (not shown) in a vertical direction depicted by arrow H for adjusting a height of the tabletop 4. Further, the tabletop 4 is tilted by a second driving device (not shown) into a Trendelenburg position or an Anti-Trendelenburg position as depicted by arrow T. Furthermore, the tabletop 4 is displaced by a third driving device (not shown) along a longitudinal axis of the tabletop 4 as depicted by arrow L. The head plate 4' is tilted by a fourth driving device (not shown) as depicted by arrow T_{H} and the leg plate 4" is tilted by a fifth driving device (not shown) as depicted by arrow T_{L}. Alternatively, the medical apparatus 3 is provided with another number of driven segments 4, 4', 4", wherein at least one driven segment 4, 4', 4" movable in different directions is provided.

The remote control 2 comprises a position sensor 5. The position sensor 5 comprises an inclinometer. Alternatively or additionally, the position sensor 5 can comprise another type of sensor, e.g., an acceleration sensors or a gyroscope combined with a geomagnetic sensor. The position sensor 5 detects a position and a motion or an acceleration of the remote control 2.

Furthermore, the remote control 2 comprises five release switches 6. The release switches 6 are respectively assigned to a specific motion of one of the driven movable segments 4, 4', 4". By a first one of the release switches 6, the control of the vertical motion of the tabletop 4 can be released, by a second one of the release switches 6, the control of the tilting of the tabletop 4 into the Trendelenburg position or Anti-Trendelenburg position can be released. A third one of the release switches 6 releases the control of the displacement of the tabletop 4 in the longitudinal direction of the tabletop 4, a fourth one of the release switches 6 releases the control of the tilting of the head plate 4', and a fifth one of the release switches 6 releases the control of the tilting of the leg plate 4". In a further alternative embodiment, another number of release switches 6, e.g., according to a number of motions of the movable segments 4, 4', 4", merely one release switch 6, or more than two release switches 6, are provided.

The remote control 2 has an elongated shape and it has an axis A parallel to the elongated shape in a X axis direction of the remote control 2 and an axis B perpendicular to the elongated shape in a Y axis direction of the remote control 2. Alternatively, the remote control 2 does not have an elongated shape and the axes A and B, if necessary, are determined by definition.

The remote control 2 further comprises a communication interface 7. The communication interface 7 establishes a communication 8 to the medical apparatus 3 in a wireless manner. Alternatively, the communication interface 7 has a connection cable and establishes the communication 8 to the medical apparatus 3 in a wired manner.

By the communication 8, the remote control 2 emits a control signal capable of controlling the motion of the movable segments 4, 4', 4" according to a motion of the remote control 2 detected by the position sensor 5 when the control of the motion of the driven movable segments 4, 4', 4" is released by actuating the release switch 6. In particular, the remote control 2 emits the control signal according to a direction of the motion of the remote control 2 detected by the position sensor 5 with respect to a position of the remote control 2 when the release switch 6 starts to release the motion. The remote control 2 emits the control signal according to an amount of the motion of the remote control 2. Additionally, the remote control 2 emits the control signal according to a speed of the motion of the remote control 2. Alternatively, the amount and/or the speed of the motion of the remote control 2 are not considered, and, further alternatively, the direction of the motion is not considered but any motion is considered for moving the segment, e.g., in a reciprocating manner.

By emitting the control signal, the remote control 2 is configured to control the direction of the motion of the driven movable segments 4, 4', 4" according to the direction of the motion of the remote control 2 with respect to a position of the remote control 2 when the release button 6 starts to release the motion. Furthermore, the remote control 2 is configured to control a speed of the motion of the driven movable segments 4, 4', 4" according to the amount of the motion of the remote control 2 with respect to the position of the remote control 2 when the release button 6 starts to release the motion, wherein the speed of the driven movable segments 4, 4', 4" is the higher the larger the amount of the motion of the remote control 2 is. Moreover, the remote control 2 is configured to control the speed of the motion of the driven movable segments 4, 4', 4" according to the speed of the motion of the remote control 2, wherein the speed of the motion of the driven movable segments 4, 4', 4" is the higher the higher the speed of the motion of the remote control 2 is. In alternative embodiments, the amount and/or the speed of the motion of the remote control 2 is not considered for the control of the speed of the driven movable segments 4, 4', 4".

The motion of the remote control 2 detected by the position sensor 5 is a tilting around the axis B of the remote control 2 perpendicular to its elongated shape and a tilting around the longitudinal axis A of the remote control 2 parallel to its elongated shape. By the detection of the motion around the axis B of the remote control 2 perpendicular to its elongated shape, the vertical motion of the tabletop 4 and the displacement motions along the axis L are controlled. By tilting the remote control 2 in one direction, the tabletop 4 is lifted and, by tilting the remote control in the other direction, the tabletop 4 is lowered. By the detection of the motion around the axis A of the remote control 2 parallel to the elongated shape, the tilting motions of the table top 4, of the head plate 4', and of the leg plate 4" are controlled. In alternative embodiments, any of the motions are controlled either by detecting the motion around the axis A of the remote control 2 or by detecting the motion around the axis B of the remote control 2.

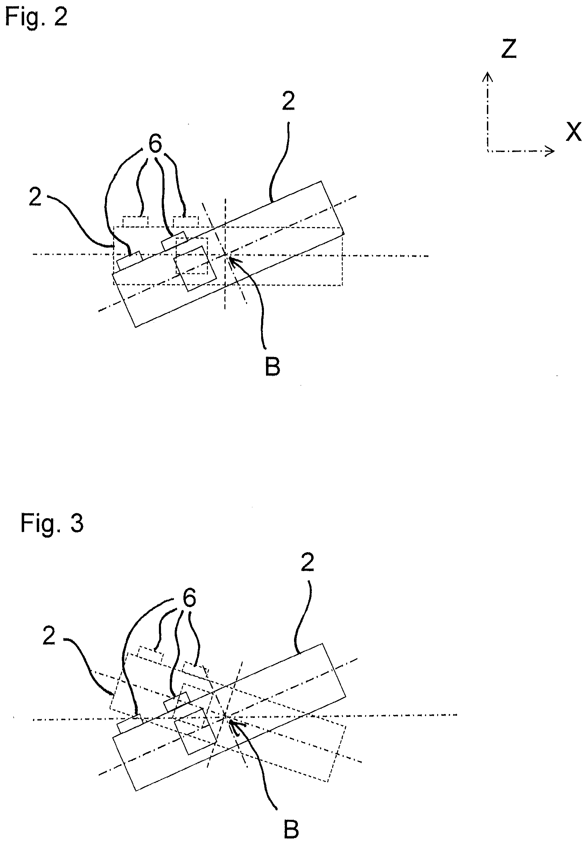

**Fig 2** shows a starting position and an end position of the remote control 2 in a side view when the remote control 2 is tilted around the axis B in the Y axis direction (Fig. 1) of the remote control 2 perpendicular to an elongated shape of the remote control 2 for controlling one of the motions. The initial position of the remote control 2 where the release switch 6 is actuated is depicted by dashed lines and the end position of the remote control 2 is depicted by solid lines. The speed and the direction of the motion of the driven movable segment 4, 4', 4", the control of which is released by actuating the respective release switch 6, are determined by the movement of the remote control 2 around the axis B. In Fig. 2, in the initial position of the remote control 2, a Z axis of the remote control 2 is oriented vertically so the remote control 2 is oriented in a horizontal position.

Fig. 3 shows another starting position and end position of the remote control 2 of Fig. 2 in the side view when the remote control 2 is tilted around the axis B perpendicular to the elongated shape of the remote control 2 for controlling one of the motions. The initial position of the remote control 2 where the release switch 6 is actuated is depicted by dashed lines and the end position of the remote control 2 is depicted by solid lines. This figure shows that the motion of the driven segment 4, 4', 4" of the medical apparatus 3 is not to be controlled compulsorily starting from the horizontal position of the remote control 2 but, also, another position is possible if the release switch 6 is actuated in this position and the remote control 2 is moved in a specific manner in order to control the motion.

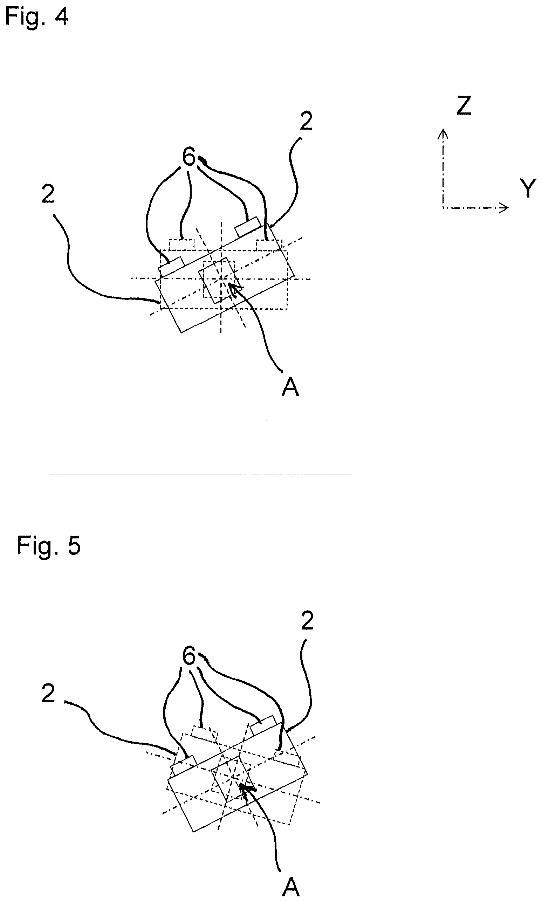

**Fig. 4** shows the starting position and the end position of the remote control 2 in a front view when the remote control 2 is tilted around the axis A in the X axis direction (Fig. 1) of the remote control 2 parallel to an elongated shape of the remote control 2. The initial position of the remote control 2 where the release switch 6 is actuated is depicted by dashed lines and the end position of the remote control 2 is depicted by solid lines. The speed and the direction of the motion of the driven movable segment 4, 4', 4", the control of which is released by actuating the respective release switch 6, are determined by the movement of the remote control 2 around the axis A. In Fig. 4, in the initial position of the remote control 2, the Z axis of the remote control 2 is oriented vertically so the remote control 2 is oriented in a horizontal position.

**Fig. 5** shows another starting position and end position of the remote control 2 of Fig. 4 in the front view when the remote control 2 is tilted around the axis A parallel to the elongated shape of the remote control 2 for controlling one of the motions. This figure shows that the motion of the driven segment 4, 4', 4" of the medical apparatus 3 is not to be controlled compulsorily starting from the horizontal position of the remote control 2 but, also, another position is possible if the release switch 6 is actuated in this position and the remote control 2 is moved in a specific manner in order to control the motion.

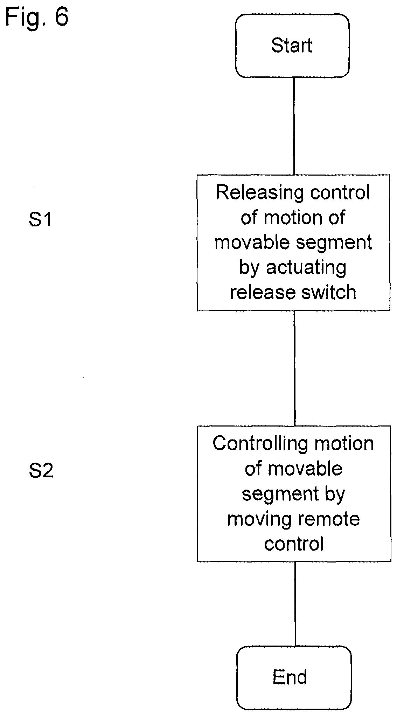

**Fig. 6** shows a flow chart of a method according to the invention.

In use, in a first step S1, the control of a specific one of the motions of the driven movable segments 4, 4', 4" of the medical apparatus 3 is released by actuating a specific release switch 6 of the remote control 2 assigned to the specific motion.

In a next step S2, the motion of the specific one of the movable segments 4, 4', 4" is controlled by moving the remote control 2 with respect to a position of the remote control 2 when the specific release switch 6 is actuated.

The direction of the motion of the specific one of the driven movable segments 4, 4', 4" is defined by the direction of motion of the remote control 2 around the axis B perpendicular to an elongated shape of the remote control 2 or around the axis A parallel to the elongated shape of the remote control 2.

The remote control 2 emits a stop command for stopping the motion of the driven movable segment 4, 4', 4" when the remote control 2 is moved into a horizontal position so that the motion of the specific one of the driven movable segments 4, 4', 4" is stopped. This function is not compulsorily and, alternatively, the motion is stopped when the release switch 6 is not actuated anymore and the release switch 6 does not release the command anymore. The horizontal position is detected by the inclinometer or, alternatively, by the acceleration sensor. The acceleration sensor detects components of acceleration forces in the directions of three axes X, Y, Z of the remote control 2. If the component of the acceleration force in the direction of the Z axis of the remote control 2 amounts 1g, the remote control 2 is determined as being oriented in the horizontal position.

While the invention has been illustrated and described in detail in the drawing and the foregoing description, such illustration and description are to be considered illustrative or exemplary and not restrictive. The invention is not limited to the disclosed embodiments. From reading the present disclosure, other modifications will be apparent to a person skilled in the art. Such modifications may involve other features, which are already known in the art and may be used instead of or in addition to features already described herein.

The invention has been described in conjunction with various embodiments herein. However, other variations to the disclosed embodiments can be understood and effected by those skilled in the art in practicing the claimed invention, from a study of the drawings, the disclosure and the appended claims. In the claims, the word "comprising" does not exclude other elements or steps, and the indefinite article "a" or "an" does not exclude a plurality.

## Claims

1. A remote control (2) for controlling a medical apparatus (3) having at least one driven movable segment (4, 4', 4"), the remote control (2) comprising:
a position sensor (5) configured to detect a position and a motion of the remote control (2);
at least one release switch (6); and
at least one respective button or softkey for actuating the or each release switch (6),
wherein the remote control (2) is configured to emit a control signal when the at least one release switch (6) is actuated, by the respective button or softkey being pressed, to release a control of the motion of the at least one driven movable segment (4, 4', 4"), wherein the control signal is capable of controlling a motion of the at least one driven movable segment (4, 4', 4") according to a motion of the remote control (2) detected by the position sensor (5), and **characterised in that** the remote control (2) is further configured to emit a stop command for stopping the motion of the at least one driven movable segment (4, 4', 4") when the remote control (2) is moved into a horizontal position.

2. The remote control (2) of claim 1, wherein
the remote control (2) is configured to emit the control signal according to a direction of the motion of the remote control (2) detected by the position sensor (5) with respect to a position of the remote control (2) when the at least one release switch (6) is actuated to start the release of the control of the motion of the at least one driven movable segment.

3. The remote control (2) of claim 1 or 2, wherein
the remote control (2) is configured to emit the control signal according to an amount of the motion of the remote control (2).

4. The remote control (2) of any one of claims 1 to 3, wherein
the remote control (2) is configured to emit the control signal according to a speed of the motion of the remote control (2).

5. The remote control (2) of any one of the preceding claims, wherein the position sensor (5) comprises an inclinometer.

6. The remote control (2) of any one of the preceding claims, wherein the position sensor (5) comprises an acceleration sensor.

7. A system (1) of a remote control (2) of any one of the preceding claims and a medical apparatus (3), wherein
the remote control (2) is configured to control the motion of at least one driven movable segment (4, 4', 4") of the medical apparatus (3) by the control signal.

8. The system (1) of claim 7, wherein
the remote control (2) is configured to control a direction of the motion of the at least one driven movable segment (4, 4', 4") according to a direction of the motion of the remote control (2) with respect to a position of the remote control (2) when the at least one release switch (6) starts to release the control of the motion.

9. The system (1) of claim 7 or 8, wherein
the remote control (2) is configured to control a speed of the motion of the at least one driven movable segment (4, 4', 4") according to an amount of the motion of the remote control (2) with respect to a position of the remote control (2) when the at least one release switch (6) starts to release the control of the motion, wherein the speed of the at least one driven movable segment (4, 4', 4") is the higher the larger the amount of the motion of the remote control (2) is.

10. The system (1) of any one of claims 7 to 9, wherein
the remote control (2) is configured to control a speed of the motion of the at least one driven movable segment (4, 4', 4") according to a speed of the motion of the remote control (2), wherein the speed of the motion of the at least one driven movable segment (4, 4', 4") is as higher as higher the speed of the motion of the remote control (2) is.

11. The system (1) of any one of claims 7 to 10, wherein
the remote control (2) comprises several release switches (6),
the medical apparatus (3) comprises at least one driven movable segment (4, 4', 4") which performs several motions respectively driven by a driving device or the medical apparatus (3) comprises several movable segments (4, 4', 4") respectively driven by a driving device, and
the several release switches (6) are respectively assigned to a specific motion of one of the driven movable segments (4, 4', 4").

12. The system (1) of any one of claims 7 to 11, wherein
the remote control (2) comprises a communication interface (7) configured to establish a communication to the medical apparatus in a wireless manner.

13. The system (1) of any one of claims 7 to 11, wherein
the remote control (2) comprises a communication interface (7) having a connection cable configured to establish a communication to the medical apparatus in a wired manner.

14. The system (1) of any one of claims 7 to 13, wherein
the medical apparatus (3) is configured as an operating table.

15. A method for operating a medical apparatus (3) by means of a remote control (2) with the steps:
releasing a control of a motion of a specific one of driven movable segments (4, 4', 4") of the medical apparatus (3) by actuating a specific release switch (6) of the remote control (2);
controlling a motion of the specific one of the movable segments (4, 4', 4") by moving the remote control (2) with respect to a position of the remote control (2) when the specific release switch is actuated, by a respective button or softkey being pressed, to release the control of the motion of the specific one of the movable segments (4, 4', 4"); and
emitting a stop command for stopping the motion of the specific one of the driven movable segments (4, 4', 4") when the remote control (2) is moved into a horizontal position.

16. The method of claim 15, wherein
a direction of the motion of the specific one of the driven movable segments (4, 4', 4") is defined by a direction of motion of the remote control (2) around an axis (B) perpendicular to an elongated shape of the remote control (2) or around an axis (A) parallel to the elongated shape of the remote control (2).

## Patentansprüche

1. Fernbedienung (2) zum Steuern einer medizinischen Vorrichtung (3), die mindestens ein angetriebenes bewegliches Segment (4, 4', 4") aufweist, wobei die Fernbedienung (2) umfasst:
einen Positionssensor (5), der so ausgebildet ist, dass er eine Position und eine Bewegung der Fernbedienung (2) erfasst;
mindestens einen Freigabeschalter (6); und
mindestens eine jeweilige Taste oder Softkey zum Betätigen des oder jedes Freigabeschalters (6),
wobei die Fernbedienung (2) so ausgebildet ist, dass sie ein Steuersignal aussendet, wenn der mindestens eine Freigabeschalter (6) durch Drücken der jeweiligen Taste oder des Softkeys betätigt wird, um eine Steuerung der Bewegung des mindestens einen angetriebenen beweglichen Segments (4, 4', 4") freizugeben, wobei das Steuersignal in der Lage ist, eine Bewegung des mindestens einen angetriebenen beweglichen Segments (4, 4', 4") entsprechend einer von dem Positionssensor (5) erfassten Bewegung der Fernbedienung (2) zu steuern, und **dadurch gekennzeichnet, dass** die Fernbedienung (2) weiter so ausgebildet ist, dass sie einen Stoppbefehl zum Stoppen der Bewegung des mindestens einen angetriebenen beweglichen Segments (4, 4', 4") aussendet, wenn die Fernbedienung (2) in eine horizontale Position bewegt wird.

2. Fernbedienung (2) nach Anspruch 1, wobei
die Fernbedienung (2) so ausgebildet ist, dass sie das Steuersignal entsprechend einer von dem Positionssensor (5) erfassten Richtung der Bewegung der Fernbedienung (2) in Bezug auf eine Position der Fernbedienung (2) aussendet, wenn der mindestens eine Freigabeschalter (6) betätigt wird, um die Freigabe der Steuerung der Bewegung des mindestens einen angetriebenen beweglichen Segments zu beginnen.

3. Fernbedienung (2) nach Anspruch 1 oder 2, wobei
die Fernbedienung (2) so ausgebildet ist, dass sie das Steuersignal entsprechend einer Menge der Bewegung der Fernbedienung (2) aussendet.

4. Fernbedienung (2) nach einem der Ansprüche 1 bis 3, wobei
die Fernbedienung (2) so ausgebildet ist, dass sie das Steuersignal entsprechend einer Geschwindigkeit der Bewegung der Fernbedienung (2) aussendet.

5. Fernbedienung (2) nach einem der vorstehenden Ansprüche, wobei der Positionssensor (5) einen Neigungsmesser umfasst.

6. Fernbedienung (2) nach einem der vorstehenden Ansprüche, wobei der Positionssensor (5) einen Beschleunigungssensor umfasst.

7. System (1) einer Fernbedienung (2) nach einem der vorstehenden Ansprüche und einer medizinischen Vorrichtung (3), wobei
die Fernbedienung (2) so ausgebildet ist, dass sie die Bewegung mindestens eines angetriebenen beweglichen Segments (4, 4', 4") der medizinischen Vorrichtung (3) durch das Steuersignal steuert.

8. System (1) nach Anspruch 7, wobei
die Fernbedienung (2) so ausgebildet ist, dass sie eine Richtung der Bewegung des mindestens einen angetriebenen beweglichen Segments (4,4',4") entsprechend einer Richtung der Bewegung der Fernbedienung (2) in Bezug auf eine Position der Fernbedienung (2) steuert, wenn der mindestens eine Freigabeschalter (6) beginnt, die Steuerung der Bewegung freizugeben.

9. System (1) nach Anspruch 7 oder 8, wobei
die Fernbedienung (2) so ausgebildet ist, dass sie eine Geschwindigkeit der Bewegung des mindestens einen angetriebenen beweglichen Segments (4, 4', 4") entsprechend einer Menge der Bewegung der Fernbedienung (2) in Bezug auf eine Position der Fernbedienung (2) steuert, wenn der mindestens eine Freigabeschalter (6) beginnt, die Steuerung der Bewegung freizugeben,
wobei die Geschwindigkeit des mindestens einen angetriebenen beweglichen Segments (4, 4', 4") umso höher ist, je größer die Menge der Bewegung der Fernbedienung (2) ist.

10. System (1) nach einem der Ansprüche 7 bis 9, wobei
die Fernbedienung (2) so ausgebildet ist, dass sie eine Geschwindigkeit der Bewegung des mindestens einen angetriebenen beweglichen Segments (4,4',4") entsprechend einer Geschwindigkeit der Bewegung der Fernbedienung (2) steuert, wobei die Geschwindigkeit der Bewegung des mindestens einen angetriebenen beweglichen Segments (4,4',4") umso höher ist, je höher die Geschwindigkeit der Bewegung der Fernbedienung (2) ist.

11. System (1) nach einem der Ansprüche 7 bis 10, wobei
die Fernbedienung (2) mehrere Freigabeschalter (6) umfasst,
die medizinische Vorrichtung (3) mindestens ein angetriebenes bewegliches Segment (4, 4', 4") umfasst, das mehrere Bewegungen durchführt, die jeweils von einer Antriebsvorrichtung angetrieben werden, oder die medizinische Vorrichtung (3) mehrere bewegliche Segmente (4, 4', 4") umfasst, die jeweils von einer Antriebsvorrichtung angetrieben werden, und
die mehreren Freigabeschalter (6) jeweils einer bestimmten Bewegung eines der angetriebenen beweglichen Segmente (4, 4', 4") zugeordnet ist.

12. System (1) nach einem der Ansprüche 7 bis 11, wobei
die Fernbedienung (2) eine Kommunikationsschnittstelle (7) umfasst, die so ausgebildet ist, dass sie eine drahtlose Kommunikation mit der medizinischen Vorrichtung herstellt.

13. System (1) nach einem der Ansprüche 7 bis 11, wobei
die Fernbedienung (2) eine Kommunikationsschnittstelle (7) umfasst, die ein Anschlusskabel aufweist, das so ausgebildet ist, dass es eine drahtgebundene Kommunikation mit der medizinischen Vorrichtung herstellt.

14. System (1) nach einem der Ansprüche 7 bis 13, wobei
die medizinische Vorrichtung (3) als Operationstisch ausgebildet ist.

15. Verfahren zum Bedienen einer medizinischen Vorrichtung (3) mittels einer Fernbedienung (2) mit den folgenden Schritten:
Freigeben einer Steuerung einer Bewegung eines bestimmten von angetriebenen beweglichen Segmenten (4, 4', 4") der medizinischen Vorrichtung (3) durch Betätigen eines bestimmten Freigabeschalters (6) der Fernbedienung (2);
Steuern einer Bewegung des spezifischen der beweglichen Segmente (4, 4', 4") durch Bewegen der Fernbedienung (2) in Bezug auf eine Position der Fernbedienung (2), wenn der spezifische Freigabeschalter durch Drücken einer jeweiligen Taste oder eines jeweiligen Softkeys betätigt wird, um die Steuerung der Bewegung des spezifischen der beweglichen Segmente (4, 4', 4") freizugeben; und
Aussenden eines Stoppbefehls zum Stoppen der Bewegung des spezifischen der angetriebenen beweglichen Segmente (4, 4', 4"), wenn die Fernbedienung (2) in eine horizontale Position bewegt wird.

16. Verfahren nach Anspruch 15, wobei
eine Richtung der Bewegung des spezifischen der angetriebenen beweglichen Segmente (4, 4', 4") durch eine Richtung der Bewegung der Fernbedienung (2) um eine Achse (B), die senkrecht zu einer länglichen Form der Fernbedienung (2) verläuft, oder um eine Achse (A), die parallel zu der länglichen Form der Fernbedienung (2) verläuft, definiert wird.

## Revendications

1. Commande à distance (2) destinée à la commande d'un appareil médical (3) présentant au moins un segment mobile entraîné (4, 4', 4"), la commande à distance (2) comprenant :
un capteur de position (5) configuré pour détecter une position et un mouvement de la commande à distance (2) ;
au moins un commutateur de déverrouillage (6) ; et
au moins un bouton ou une touche respectif pour actionner le ou chaque commutateur de déverrouillage (6),
la commande à distance (2) étant configurée pour émettre un signal de commande lorsque l'au moins un commutateur de déverrouillage (6) est actionné, par pression du bouton ou de la touche respectif, afin de déverrouiller une commande du mouvement de l'au moins un segment mobile entraîné (4, 4', 4"), dans laquelle le signal de commande est capable de commander un mouvement de l'au moins un segment mobile entraîné (4, 4', 4") conformément à un mouvement de la commande à distance (2) détecté par le capteur de position (5), et **caractérisée en ce que** la commande à distance (2) est configurée en outre pour émettre une commande d'arrêt afin d'arrêter le mouvement de l'au moins un segment mobile entraîné (4, 4', 4") lorsque la commande à distance (2) est mise en position horizontale.

2. Commande à distance (2) selon la revendication 1, dans laquelle
la commande à distance (2) est configurée pour émettre le signal de commande conformément à une direction du mouvement de la commande à distance (2) détecté par le capteur de position (5) par rapport à la position de la commande à distance (2) lorsque l'au moins un commutateur de déverrouillage (6) est actionné pour commencer le déverrouillage de la commande du mouvement de l'au moins un segment mobile entraîné.

3. Commande à distance (2) selon la revendication 1 ou 2, dans laquelle
la commande à distance (2) est configurée pour émettre le signal de commande conformément à une quantité de mouvement de la commande à distance (2).

4. Commande à distance (2) selon l'une quelconque des revendications 1 à 3, dans laquelle
la commande à distance (2) est configurée pour émettre le signal de commande conformément à une vitesse du mouvement de la commande à distance (2).

5. Commande à distance (2) selon l'une quelconque des revendications précédentes, dans laquelle
le capteur de position (5) comprend un inclinomètre.

6. Commande à distance (2) selon l'une quelconque des revendications précédentes, dans laquelle
le capteur de position (5) comprend un capteur d'accélération.

7. Système (1) d'une commande à distance (2) selon l'une quelconque des revendications précédentes et d'un appareil médical (3), dans lequel
la commande à distance (2) est configurée pour commander le mouvement d'au moins un segment mobile entraîné (4, 4', 4") de l'appareil médical (3) par le signal de commande.

8. Système (1) selon la revendication 7, dans lequel
la commande à distance (2) est configurée pour commander une direction du mouvement de l'au moins un segment mobile entraîné (4, 4', 4"") conformément à une direction du mouvement de la commande à distance (2) par rapport à la position de la commande à distance (2) lorsque l'au moins un commutateur de déverrouillage (6) commence à déverrouiller la commande du mouvement.

9. Système (1) selon la revendication 7 ou 8, dans lequel
la commande à distance (2) est configurée pour commander une vitesse du mouvement de l'au moins un segment mobile entraîné (4, 4', 4") conformément à une quantité du mouvement de la commande à distance (2) par rapport à la position de la commande à distance (2) lorsque l'au moins un commutateur de déverrouillage (6) commence à déverrouiller la commande du mouvement, dans lequel plus le mouvement de la commande à distance (2) est important, plus la vitesse de l'au moins un segment mobile entraîné (4, 4', 4") est élevée.

10. Système (1) selon l'une quelconque des revendications 7 à 9, dans lequel
la commande à distance (2) est configurée pour commander une vitesse du mouvement de l'au moins un segment mobile entraîné (4, 4', 4") conformément à une vitesse du mouvement de la commande à distance (2), dans lequel la vitesse du mouvement de l'au moins un segment mobile entraîné (4, 4', 4") est aussi élevée que la vitesse du mouvement de la commande à distance (2).

11. Système (1) selon l'une quelconque des revendications 7 à 10, dans lequel
la commande à distance (2) comprend plusieurs interrupteurs de déverrouillage (6),
l'appareil médical (3) comprend au moins un segment mobile entraîné (4, 4', 4") qui effectue plusieurs mouvements respectivement entraînés par un dispositif d'entraînement ou l'appareil médical (3) comprend plusieurs segments mobiles (4, 4', 4") respectivement entraînés par un dispositif d'entraînement, et
les plusieurs commutateurs de libération (6) sont respectivement attribués à un mouvement spécifique de l'un des segments mobiles entraînés (4, 4', 4").

12. Système (1) selon l'une quelconque des revendications 7 à 11, dans lequel
la commande à distance (2) comprend une interface de communication (7) configurée pour établir une communication sans fil avec l'appareil médical.

13. Système (1) selon l'une quelconque des revendications 7 à 11, dans lequel
la commande à distance (2) comprend une interface de communication (7) avec un câble de connexion configuré pour établir une communication filaire avec l'appareil médical.

14. Système (1) selon l'une quelconque des revendications 7 à 13, dans lequel
l'appareil médical (3) est configuré comme une table d'opération.

15. Procédé d'exploitation d'un appareil médical (3) au moyen d'une commande à distance (2) comportant les étapes suivantes :
le déverrouillage d'une commande d'un mouvement spécifique d'un segment mobile entraîné (4, 4', 4") de l'appareil médical (3) en actionnant un commutateur de déverrouillage spécifique (6) de la commande à distance (2) ;
la commande d'un mouvement du segment spécifique des segments mobiles (4, 4', 4") en déplaçant la commande à distance (2) par rapport à une position de la commande à distance (2) lorsque le commutateur de déverrouillage spécifique est actionné, par pression d'un bouton ou d'une touche respectif, pour déverrouiller la commande du mouvement du segment spécifique des segments mobiles (4, 4', 4") ; et
l'émission d'une commande d'arrêt pour arrêter le mouvement du segment spécifique des segments mobiles entraînés (4, 4', 4") lorsque la commande à distance (2) est mise en position horizontale.

16. Procédé selon la revendication 15, dans lequel
une direction du mouvement du segment spécifique des segments mobiles entraînés (4, 4', 4") est définie par une direction de mouvement de la commande à distance (2) autour d'un axe (B) perpendiculaire à une forme allongée de la commande à distance (2) ou autour d'un axe (A) parallèle à la forme allongée de la commande à distance (2).
